# DEMANDE DE BREVET EUROPEEN

(11) **EP 3 875 029 A1**
(43) Date de publication de la demande: **08.09.2021**
(21) Numéro de dépôt: 20160412.1
(22) Date de dépôt: 02.03.2020
(51) Int. Cl.: A61B 5/157, C12M 3/06, C12Q 1/6825

(54) **DISPOSITIF POUR DÉTECTER LA PRÉSENCE D'ADN CIBLE DANS UN ÉCHANTILLON, EN PARTICULIER DANS UN ÉCHANTILLON LIQUIDE**

(71) Demandeur: MAGNETRAP, 7000 Mons (BE); UNIVERSITE CLAUDE BERNARD - LYON 1, 69100 Villeurbanne (FR); Institut National des Sciences Appliquées de Lyon, 69621 Villeurbanne (FR); Ecole Supérieure de Chimie Physique Electronique de Lyon, 69616 Villeurbanne (FR); Centre National de la Recherche Scientifique, 75794 Paris Cedex 16 (FR)
(72) Inventeur: PICOT, Stéphane, 38170 Seyssinet-Pariset (FR); CLEMENT, Romain, 69007 Lyon (FR); DOUMECHE, Bastien, 69004 Lyon (FR); BIENVENU, Anne-Lise, 69006 Lyon (FR); MONIOTTE, Nicolas, 1490 Court-Saint-Etienne (BE); DERENNE, Aline, 7050 Herchies (BE); HUBINON, François, 5101 Namur (BE); CHATELLIER, Sonia, 59223 Roncq (FR)
(74) Mandataire: ABYOO

(57) **Abrégé**

La présente invention porte sur un dispositif pour détecter la présence d'ADN cible dans un échantillon, en particulier dans un échantillon liquide.

## Description

La présente invention porte sur un dispositif pour détecter la présence d'ADN cible dans un échantillon, en particulier dans un échantillon liquide.

Un dispositif selon l'invention trouve de nombreuses applications dans les domaines de la santé, notamment pour détecter, chez un individu au départ duquel est simplement prélevé un échantillon, la présence d'ADN cible indiquant que l'individu souffre d'une pathologie ou d'une maladie donnée. Il peut par exemple s'agir d'une pathologie ou d'une maladie virale (hépatites virales, ...) ou d'une pathologie ou d'une maladie bactérienne (méningite, ...) ou encore d'une pathologie ou d'une maladie parasitaire (paludisme, ...).

Il existe de nombreux dispositifs pour détecter la présence d'ADN cible dans un échantillon, notamment dans un échantillon liquide. Des dispositifs de détection d'ADN cible sont connus dont certains permettent la réalisation d'une amplification d'ADN cible pour obtenir des amplicons de l'ADN cible (par exemple par amplification isothermique à médiation par boucles - LAMP -), en présence d'un agent intercalant d'ADN présentant des propriétés oxydo-réductrices : on parle alors de mesures électrochimiques utilisant un indicateur redox. Un tel dispositif connu de l'état de la technique peut être constitué d'un réceptacle destiné à contenir un échantillon prétraité et étant associé à des électrodes permettant la réalisation de mesures de type électrochimique au sein de l'échantillon prétraité afin d'y détecter la présence d'ADN cible.

Typiquement, avec les dispositifs actuels, avant de placer un échantillon prélevé chez un individu dans un tel réceptacle et donc de pouvoir procéder à une mesure de type électrochimique, l'échantillon doit être prétraité, c'est-à-dire qu'il doit être mis en solution en présence de réactifs permettant de réaliser une amplification dudit ADN cible et en présence de réactifs de type agent intercalant d'ADN présentant des propriétés oxydo-réductrices. En pratique, l'agent intercalant d'ADN interagit à la fois avec les amplicons obtenus et avec les électrodes pour permettre d'effectuer une mesure électrochimique et de déterminer si l'échantillon prélevé chez l'individu contient ou non l'ADN cible et donc de déterminer si l'individu souffre d'une pathologie ou d'une maladie donnée (ciblée).

Malheureusement, si de tels dispositifs sont adaptés à des analyses réalisées en laboratoire, ils sont peu adéquats voire totalement inadaptés pour une détection d'ADN cible devant être effectuée sur le terrain. En effet, puisque le prétraitement d'un échantillon prélevé chez un individu requiert toujours la mise en œuvre de nombreux réactifs (dont les réactifs permettant de réaliser une amplification de l'ADN cible et l'agent intercalant), l'opérateur doit non seulement disposer de matériel (réactifs, solvants, pipettes, dispositif de chauffage, ...) mais aussi réaliser de nombreuses manipulations délicates comme par exemple des dilutions qui se doivent d'être précises. Il est évident que ceci est particulièrement contraignant sur le terrain et qu'il est même parfois tout simplement impossible d'assurer une préparation correcte de l'échantillon prélevé, ce qui peut mener à des mesures électrochimiques erronées (faux négatifs et/ou présence de contaminants par exemple). Par ailleurs, les réactifs mis en œuvre sont généralement sensibles aux conditions environnementales (températures élevées, humidité, ...) et/ou dangereux à manipuler, ce qui rend d'autant plus contraignantes les détections d'ADN cible effectuées sur le terrain.

Il existe donc actuellement un réel besoin de procurer un dispositif pour détecter la présence d'ADN cible dans un échantillon, en particulier dans un échantillon liquide, qui permette de s'affranchir au moins partiellement de ces problématiques lorsqu'une détection d'un ADN cible nécessite d'être effectuée, en particulier lorsqu'elle nécessite d'être effectuée sur le terrain.

Pour résoudre ces problèmes, il est prévu, suivant l'invention, un dispositif pour détecter la présence d'ADN cible dans un échantillon, en particulier dans un échantillon liquide, ledit dispositif comprenant :
- des réactifs permettant la réalisation d'une amplification dudit ADN cible pour obtenir des amplicons de l'ADN cible, par exemple la réalisation d'une amplification isothermique à médiation par boucles (LAMP) dudit ADN cible pour obtenir des amplicons de l'ADN cible ;
- un agent intercalant d'ADN présentant des propriétés oxydo-réductrices et pouvant s'intercaler entre des molécules desdits amplicons, en particulier pouvant s'intercaler dans un espace compris entre deux paires de bases desdits amplicons ;

- un élément chauffant électrique agencé pour chauffer ledit échantillon pendant que ce dernier est en contact ou après que ce dernier ait été en contact avec lesdits réactifs permettant la réalisation de l'amplification dudit ADN cible pour obtenir des amplicons de l'ADN cible, par exemple la réalisation d'une amplification isothermique à médiation par boucles (LAMP) ; et
- des électrodes agencées pour être en contact avec ledit échantillon pendant que ce dernier est en contact ou après que ce dernier ait été en contact avec lesdits réactifs permettant la réalisation de l'amplification dudit ADN cible pour obtenir des amplicons de l'ADN cible, par exemple la réalisation d'une amplification isothermique à médiation par boucles (LAMP), et avec ledit agent intercalant d'ADN.

Selon l'invention, de préférence, lesdites électrodes sont en outre agencées pour être accessibles électriquement depuis l'extérieur dudit dispositif.

Avec un tel dispositif de détection d'ADN cible selon invention, l'échantillon prélevé chez un individu ne nécessite pas de prétraitement, seule une lyse de l'échantillon étant parfois mais non indispensablement nécessaire selon certains modes de réalisation suivant l'invention, le dispositif selon l'invention comprenant intrinsèquement les réactifs permettant la réalisation d'une amplification dudit ADN cible pour obtenir des amplicons de l'ADN cible, l'agent intercalant d'ADN et un élément chauffant électrique.

Le dispositif selon l'invention permet ainsi à l'opérateur de se dispenser d'une quantité importante de matériel et de réactifs mais aussi de réduire considérablement les manipulations à effectuer sur le terrain pour détecter un ADN cible au départ d'un échantillon prélevé chez un individu. Il en résulte notamment que les erreurs de manipulation et les contaminations sont significativement réduites, que les mesures électrochimiques réalisées sont d'autant plus fiables et que le temps requis pour réaliser une détection sur le terrain est significativement réduit. En outre, un dispositif de détection d'un ADN cible selon l'invention est peu coûteux et peu encombrant puisqu'il ne nécessite pas d'appareillages imposants tels que ceux utilisés en laboratoire.

Les techniques d'amplification de l'ADN sont bien connues de l'homme de métier. En particulier, la technique d'amplification isothermique à médiation par boucles (LAMP) d'ADN cible est une technique bien connue de l'homme de métier qui est dès lors a même de déterminer, pour un ADN cible donné, quels réactifs doivent être compris dans le dispositif selon l'invention (tampons, dNTPs, polymérase, ...).

Selon l'invention, l'agent intercalant d'ADN présentant des propriétés oxydo-réductrices peut être du bleu de méthylène, du vert de méthylène, du bleu de crésyle brillant, du bleu de toluidine ou encore de l'acétate de thionine. Cette liste est non exhaustive et tout autre agent intercalant d'ADN adéquat tombe sous le couvert de la présente invention.

De préférence, le dispositif selon l'invention présente :
- une première partie ou zone comprenant lesdits réactifs permettant la réalisation d'une amplification dudit ADN cible pour obtenir des amplicons de l'ADN cible, par exemple la réalisation d'une amplification isothermique à médiation par boucles (LAMP) ; et
- une deuxième partie ou zone comprenant ledit agent intercalant d'ADN présentant des propriétés oxydo-réductrices et pouvant s'intercaler entre des molécules desdits amplicons.

Une distinction entre ces deux parties ou zones permet de séquencer les étapes d'amplification dudit ADN cible pour obtenir des amplicons de l'ADN cible (par exemple par amplification isothermique à médiation par boucles - LAMP -) et d'intercalation, ce qui permet au dispositif selon l'invention d'être optimal, des amplicons étant d'abord obtenus avant qu'ils n'entrent en contact avec l'agent intercalant.

Avantageusement, le dispositif selon l'invention comprend une vanne située entre ladite première partie ou zone et ladite deuxième partie ou zone. La présence d'une vanne permet de commander le passage de l'échantillon lysé ou non depuis la première partie vers la deuxième partie après une durée déterminée de séjour de l'échantillon dans la première partie de telle sorte que l'amplification dudit ADN cible pour obtenir des amplicons de l'ADN cible (par exemple par amplification isothermique à médiation par boucles - LAMP -) est optimisée.

Préférentiellement, le dispositif selon l'invention comprend en outre des réactifs pour lyser ledit échantillon ou une solution pour lyser ledit échantillon.

Avantageusement, le dispositif selon l'invention présente une partie ou zone additionnelle comprenant lesdits réactifs pour lyser ledit échantillon ou ladite solution pour lyser ledit échantillon, ladite partie ou zone additionnelle étant située en amont de ladite première partie et de ladite deuxième partie dans un sens d'écoulement/de déplacement de l'échantillon.

Selon le type d'échantillon prélevé au départ d'un individu, il peut s'avérer utile de procéder à une lyse de l'échantillon. Afin d'éviter que l'opérateur n'ait a effectué une lyse sur le terrain avec du matériel spécifique et encombrant (pipette, solution de lyse, ...), le dispositif selon l'invention peut avantageusement comprendre une chambre de lyse (partie ou zone additionnelle) dans laquelle l'échantillon est lysé avant de gagner les autres parties ou zones du dispositif.

De préférence, le dispositif selon l'invention comprend une vanne située entre ladite partie ou zone additionnelle et ladite première partie ou zone et/ou ladite deuxième partie ou zone. La présence d'une telle vanne permet de commander le passage depuis la partie ou zone additionnelle où a lieu la lyse de l'échantillon vers la première et/ou la deuxième partie après une durée déterminée de séjour de l'échantillon dans cette partie ou zone additionnelle de telle sorte que la lyse de l'échantillon est optimisée.

Selon un mode de réalisation préféré du dispositif suivant l'invention, lesdits réactifs permettant la réalisation d'une amplification dudit ADN cible pour obtenir des amplicons de l'ADN cible (par exemple la réalisation d'une amplification isothermique à médiation par boucles) et/ou ledit agent intercalant d'ADN présentant des propriétés oxydo-réductrices et pouvant s'intercaler entre des molécules desdits amplicons sont sous forme anhydre ou lyophilisée. Au contraire de solutions liquides comprenant les réactifs permettant la réalisation d'une amplification dudit ADN cible et/ou l'agent intercalant, une forme anhydre ou lyophilisée de ces derniers permet de disposer d'un dispositif qui peut être conservé plusieurs mois voire plusieurs années sans dégradations des réactifs et/ou de l'agent intercalant, lesquels conservent leurs propriétés au cours du temps, ce qui contribue à une reproductibilité des résultats.

De façon préférentielle selon l'invention, lesdits réactifs permettant la réalisation d'une amplification dudit ADN cible pour obtenir des amplicons de l'ADN cible et/ou ledit agent intercalant d'ADN présentant des propriétés oxydo-réductrices et/ou lesdits réactifs pour lyser ledit échantillon se présentent sous la forme d'un lyophilisat dans le dispositif suivant l'invention.

Selon un mode de réalisation avantageux du dispositif suivant l'invention, ledit agent intercalant et/ou lesdits réactifs permettant la réalisation d'une amplification dudit ADN cible pour obtenir des amplicons de l'ADN cible, par exemple la réalisation d'une amplification isothermique à médiation par boucles sont présents sur un support, par exemple sur un support cellulosique, comportant des charges positives et/ou des groupements chargés positivement et/ou des polymères chargés positivement. Par exemple, des groupements chargés positivement sont associés à un support, en particulier à un support cellulosique, par greffage covalent ou autre. Alternativement ou complémentairement, des polymères chargés positivement sont associés à un support, en particulier à un support cellulosique, par adsorption. Alternativement ou complémentairement, des espèces chimiques prédéterminées sont polymérisées en surface d'un support, en particulier en surface d'un support cellulosique, pour que des polymères chargés positivement soient associés à ce support. Dans le cadre de la présente invention, il a été mis en avant, en particulier pour l'agent intercalant (par exemple pour le bleu de méthylène), qu'un tel support chargé positivement permet de garantir une disponibilité adéquate de l'agent intercalant, en ce sens qu'il ne reste pas accroché au support et qu'il peut ainsi s'intercaler entre les molécules des amplicons obtenus afin d'assurer une mesure électrochimique adéquate.

Avantageusement, selon l'invention ledit échantillon est choisi parmi un échantillon sanguin, un échantillon salivaire, un échantillon urinaire. L'échantillon peut se présenter sous forme liquide ou semi-liquide mais aussi sous forme solide ou semi-solide.

De préférence, selon l'invention, ledit élément chauffant électrique comprend une résistance électrique, laquelle est alimentée par une source d'énergie électrique.

Préférentiellement, selon l'invention, lesdites électrodes sont au nombre de trois.

De préférence, le dispositif selon l'invention se présente sous la forme d'une cartouche ou d'un dispositif microfluidique, par exemple sous forme d'une cellule ou d'une puce microfluidique ou d'un dispositif microfluidique à base de papier.

Avantageusement, le dispositif selon l'invention se présente sous la forme d'une cartouche au travers de laquelle peut s'écouler une solution ou d'un dispositif microfluidique, par exemple sous forme d'une cellule ou d'une puce microfluidique. Le dispositif selon l'invention peut également se présenter sous forme d'un dispositif microfluidique à base de papier (« paper-based microfluidics ») consistant par exemple en une série de fibres de cellulose ou de nitrocellulose hydrophiles qui guident un liquide d'une entrée à une zone souhaitée par imbibition et/ou par capillarité. Eventuellement, le dispositif microfluidique à base de papier selon l'invention se présente sous la forme d'un origami (« paper-origami based »), c'est-à-dire sous la forme d'un papier microfluidique dont des parties sont prévues pour être pliées l'une sur l'autre ou les unes sur les autres pour être mises en contact. Au sens de la présente invention, le dispositif peut préférentiellement encore se présenter sous la forme d'un « paper-based point-of-care NAATs (Nucleic acid amplification-based tests) ».

La présente invention porte également sur un ensemble comprenant :
- un dispositif selon l'invention ; et
- un appareil ou un appareillage pouvant être relié électriquement aux électrodes pour effectuer des mesures électrochimiques, de préférence des mesures électrochimiques voltampérométriques, plus préférentiellement des mesures électrochimiques voltampérométriques cycliques ou à ondes carrées.

Typiquement, pour réaliser des mesures électrochimiques, de préférence des mesures électrochimiques voltampérométriques, plus préférentiellement des mesures électrochimiques voltampérométriques cycliques ou à ondes carrées, l'appareillage comprend un potentiostat pour appliquer une différence de potentiel entre les électrodes et/ou une unité de traitement de signaux émis par le potentiostat ou reçus par le potentiostat.

La présente invention porte encore sur l'utilisation d'un dispositif selon l'invention ou d'un ensemble selon l'invention pour détecter la présence d'ADN cible dans un échantillon.

D'autres caractéristiques, détails et avantages de l'invention ressortiront des exemples donnés ci-après, à titre non limitatif et en faisant référence aux figures annexées. Généralement, sur les figures, les éléments similaires portent la même référence.
La figure 1 illustre un premier exemple d'un mode de réalisation d'un dispositif selon l'invention.
La figure 2 illustre un second exemple d'un mode de réalisation d'un dispositif selon l'invention.
La figure 3 illustre un troisième exemple d'un mode de réalisation d'un dispositif selon l'invention.
La figure 4 illustre un quatrième exemple d'un mode de réalisation d'un dispositif selon l'invention.
Les figures 5A, 5B et 5C sont des vues éclatées d'exemples de modes de réalisation de dispositifs selon l'invention et illustrent les composants de différents modes de réalisation d'un dispositif selon l'invention.
Les figures 6A, 6B, 6C, 6D et 6E sont des vues éclatées d'exemples de modes de réalisation de dispositifs selon l'invention et illustrent également les composants de différents modes de réalisation d'un dispositif selon l'invention.
Les figures 7A, 7B, 7C et 7D illustrent encore des exemples de modes de réalisation de dispositifs selon l'invention.

### Exemples

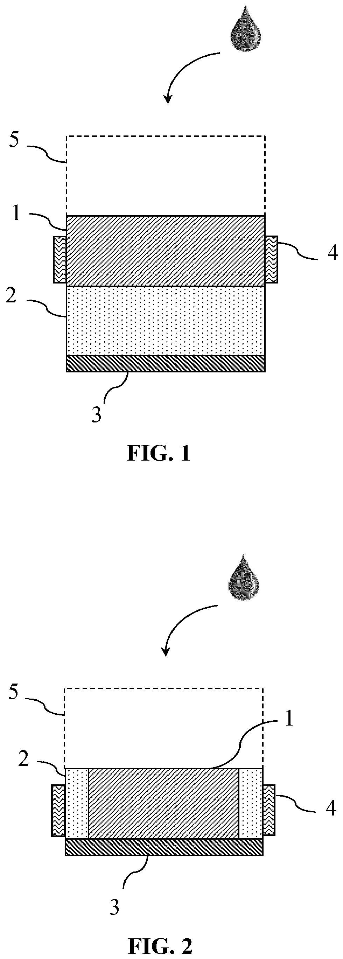

La figure 1 illustre un premier mode de réalisation d'un dispositif selon l'invention qui, comme illustré comprend une première partie ou zone 1, une deuxième partie ou zone 2, des électrodes 3 et un élément chauffant électrique 4. Optionnellement, comme illustré par les pointillés, ce mode de réalisation d'un dispositif selon l'invention peut comprendre une partie ou zone additionnelle 5 comprenant des réactifs pour lyser l'échantillon ou une solution pour lyser un échantillon prélevé au départ d'un individu. La figure 1 illustre un exemple d'un dispositif selon l'invention qui pourrait se présenter sous la forme d'une cartouche ou d'un dispositif microfluidique, par exemple sous la forme d'un dispositif microfluidique à base de papier (« paper-based microfluidics » ou « paper-origami based » ou « paper-based point-of-care NAATs »).

La première partie ou zone 1 comprend des réactifs permettant la réalisation d'une amplification dudit ADN cible pour obtenir des amplicons de l'ADN cible potentiellement contenu dans l'échantillon (par exemple la réalisation d'une amplification isothermique à médiation par boucles) pour obtenir des amplicons de l'ADN cible potentiellement contenu dans l'échantillon. La deuxième partie ou zone 2 comprend un agent intercalant d'ADN présentant des propriétés oxydo-réductrices et pouvant s'intercaler entre des molécules des amplicons, en particulier pouvant s'intercaler dans l'espace compris entre deux paires de bases des amplicons.

Avec un tel dispositif selon l'invention, l'échantillon prélevé au départ d'un individu est de préférence lysé, c'est-à-dire mis en solution dans une solution de lyse, avant d'être introduit dans le dispositif comme indiqué par la flèche. Lorsque, par exemple sous l'effet de la gravité, l'échantillon lysé gagne la première partie ou zone 1, il entre en contact avec les réactifs permettant la réalisation d'une amplification dudit ADN cible pour obtenir des amplicons de l'ADN cible (par exemple la réalisation d'une amplification isothermique à médiation par boucles), dans la première partie ou zone 1 sous l'effet d'un chauffage à une température prédéterminée par l'élément chauffant électrique 4. Des amplicons de l'ADN cible sont ainsi obtenus à condition que cet ADN cible soit présent dans l'échantillon prélevé au départ de l'individu. Les amplicons obtenus gagnent alors, par exemple sous l'effet de la gravité, la deuxième partie ou zone 2 comprenant un agent intercalant d'ADN, lequel s'intercale entre les molécules des amplicons, en particulier entre deux paires de bases des amplicons.

Selon le mode de réalisation illustré, les électrodes 3 (électrode de travail, contre-électrode ou électrode auxiliaire et électrode de référence) sont en contact avec l'échantillon après que ce dernier ait été en contact avec lesdits réactifs permettant la réalisation de l'amplification dudit ADN cible pour obtenir des amplicons de l'ADN cible (par exemple la réalisation d'une amplification isothermique à médiation par boucles), et avec ledit agent intercalant d'ADN. Les électrodes 3 sont également accessibles électriquement depuis l'extérieur du dispositif selon l'invention de telle sorte qu'elles peuvent être reliées électriquement à un appareillage (non illustré) permettant de réaliser des mesures électrochimiques (de préférence des mesures électrochimiques voltampérométriques, plus préférentiellement des mesures électrochimiques voltampérométriques cycliques ou à ondes carrées) utilisant un indicateur redox (agent intercalant), le principe et la mise en œuvre d'une telle mesure (AND/amplicons + agent intercalant ou agent redox) étant bien connus de l'homme de métier.

Lorsque le mode de réalisation illustré à la figure 1 comprend également une partie ou zone additionnelle 5 comprenant une solution ou des réactifs pour lyser un échantillon prélevé au départ d'un individu, l'échantillon peut être directement introduit dans le dispositif au niveau de la partie ou zone additionnelle 5 dans laquelle l'échantillon est lysé avant de gagner, par exemple sous l'effet de la gravité, la première partie ou zone 1 puis la deuxième partie ou zone 2.

La figure 2 illustre un second mode de réalisation d'un dispositif selon l'invention. La figure 2 illustre un exemple d'un dispositif selon l'invention qui pourrait se présenter sous la forme d'une cartouche ou d'un dispositif microfluidique, par exemple sous la forme d'un dispositif microfluidique à base de papier (« paper-based microfluidics » ou « paper-origami based » ou « paper-based point-of-care NAATs »). Les mêmes éléments que ceux illustrés à la figure 1 sont repris. Toutefois, selon le mode de réalisation tel qu'illustré à la figure 2, la première partie ou zone 1 et la deuxième partie ou zone 2 sont situées à un même niveau, c'est-à-dire dans une même zone ou un même compartiment. Par exemple, l'agent intercalant situé dans la deuxième partie ou zone 2 peut revêtir les parois latérales d'une zone ou d'un compartiment du dispositif en entourant ainsi la première partie ou zone 1 comprenant les réactifs permettant la réalisation de l'amplification dudit ADN cible pour obtenir des amplicons de l'ADN cible potentiellement contenu dans l'échantillon (par exemple la réalisation d'une amplification isothermique à médiation par boucles). Il n'est pas exclu, selon l'invention, que les réactifs permettant la réalisation de l'amplification dudit ADN cible et que ledit agent intercalant soit présent en mélange dans une même zone ou dans un même compartiment, lequel comprendrait alors ladite première et ladite deuxième partie ou zone.

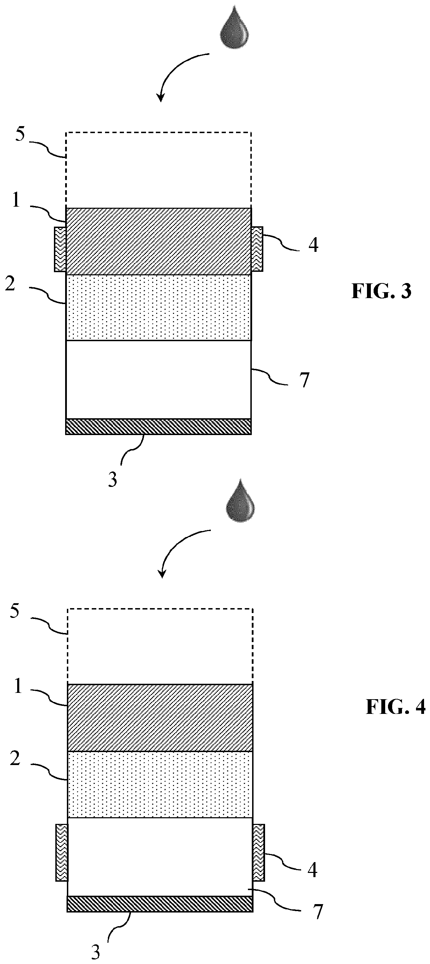

La figure 3 illustre un troisième mode de réalisation d'un dispositif selon l'invention. La figure 3 illustre un exemple d'un dispositif selon l'invention qui pourrait se présenter sous la forme d'une cartouche ou d'un dispositif microfluidique, par exemple sous la forme d'un dispositif microfluidique à base de papier (« paper-based microfluidics » ou « paper-origami based » ou « paper-based point-of-care NAATs »). A la différence du mode de réalisation illustré à la figure 1, celui illustré à la figure 3 comprend une partie ou zone 7 supplémentaire située en amont et en contact avec les électrodes 3 dans un sens d'écoulement de l'échantillon.

Le mode de réalisation illustré à la figure 4 est identique à celui de la figure 3 hormis le fait que le chauffage assuré par l'élément chauffant électrique 4 s'effectue au niveau de la partie ou zone 7 supplémentaire située en amont et en contact avec les électrodes 3 dans un sens d'écoulement de l'échantillon plutôt qu'au niveau de la première partie ou zone 1. La figure 4 illustre un exemple d'un dispositif selon l'invention qui pourrait se présenter sous la forme d'une cartouche ou d'un dispositif microfluidique, par exemple sous la forme d'un dispositif microfluidique à base de papier (« paper-based microfluidics » ou « paper-origami based » ou « paper-based point-of-care NAATs »).

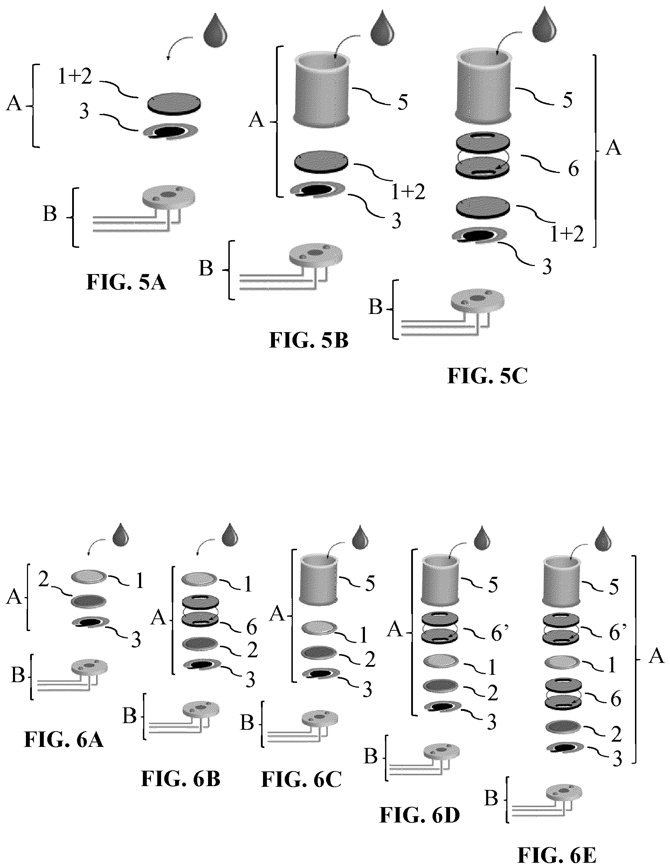

Les figures 5A, 5B et 5C illustrent les composants de différents modes de réalisation d'un dispositif selon l'invention. Plus particulièrement, les figures 5A, 5B et 5C illustrent les composants d'un dispositif A selon l'invention qui se présente sous la forme d'un cylindre constituant par exemple une cartouche. A la figure 5A, le dispositif A selon l'invention comprend une première partie constituée par exemple d'un disque perméable 1+2, par exemple d'un disque perméable en matière cellulosique, comprenant à la fois les réactifs permettant la réalisation de l'amplification dudit ADN cible pour obtenir des amplicons de l'ADN cible (par exemple la réalisation d'une amplification isothermique à médiation par boucles), et l'agent intercalant d'ADN. Un élément chauffant électrique (non illustré) assure un chauffage du disque perméable 1+2 à une température prédéterminée pour assurer l'amplification dudit ADN cible pour obtenir des amplicons de l'ADN cible (par exemple une amplification isothermique à médiation par boucles). Selon le mode de réalisation de la figure 5A, des électrodes 3 au nombre de trois sont en contact avec l'échantillon pendant que ce dernier est en contact avec lesdits réactifs permettant la réalisation de l'amplification dudit ADN cible pour obtenir des amplicons de l'ADN cible (par exemple la réalisation d'une amplification isothermique à médiation par boucles), et avec ledit agent intercalant d'ADN. En outre, les électrodes 3 sont également accessibles électriquement depuis l'extérieur du dispositif selon l'invention de telle sorte qu'elles peuvent être reliées électriquement à un appareillage (non illustré) permettant de réaliser des mesures électrochimiques (de préférence des mesures électrochimiques voltampérométriques, plus préférentiellement des mesures électrochimiques voltampérométriques cycliques ou à ondes carrées) utilisant un indicateur redox (agent intercalant), le principe et la mise en œuvre d'une telle mesure (ADN/amplicons + agent intercalant) étant bien connus de l'homme de métier. Comme illustré à la figure 5A, les électrodes 3 du dispositif A selon l'invention peuvent être reliées électriquement à un appareillage (non illustré) par l'intermédiaire d'une base B ou d'un socle muni de connecteurs. Par exemple, le dispositif A selon l'invention peut être visé ou clipsé sur le socle ou la base B pour assurer un contact adéquat entre les connecteurs et les électrodes 3.

La figure 5B est identique à la figure 5A mais le dispositif A illustré comprend en plus une partie ou zone additionnelle 5 comprenant des réactifs ou une solution pour lyser un échantillon prélevé au départ d'un individu. De la sorte, l'échantillon peut être directement introduit dans le dispositif au niveau de la partie ou zone additionnelle 5 dans laquelle l'échantillon est lysé avant de gagner, par exemple sous l'effet de la gravité, la partie constituée par exemple d'un disque perméable 1+2 comprenant à la fois les réactifs permettant la réalisation de l'amplification dudit ADN cible pour obtenir des amplicons de l'ADN cible (par exemple la réalisation d'une amplification isothermique à médiation par boucles), et l'agent intercalant d'ADN.

La figure 5C est identique à la figure 5B mais le dispositif A illustré comprend en plus une vanne 6, par exemple une vanne à tiroir rotative, permettant de commander le passage de l'échantillon depuis la partie ou zone additionnelle 5 où il est lysé vers la partie constituée par exemple d'un disque perméable 1+2 comprenant à la fois les réactifs permettant la réalisation de l'amplification dudit ADN cible pour obtenir des amplicons de l'ADN cible (par exemple la réalisation d'une amplification isothermique à médiation par boucles), et l'agent intercalant d'ADN.

Les figures 6A, 6B, 6C, 6D et 6E illustrent également les composants de différents modes de réalisation d'un dispositif selon l'invention. Plus particulièrement, les figures 6A, 6B, 6C, 6D et 6E illustrent les composants d'un dispositif A selon l'invention qui se présente sous la forme d'un cylindre constituant par exemple une cartouche. A la figure 6A, le dispositif A selon l'invention comprend :
- une première partie 1 constituée par exemple d'un disque perméable, par exemple d'un disque perméable en matière cellulosique, comprenant les réactifs permettant la réalisation de l'amplification dudit ADN cible pour obtenir des amplicons de l'ADN cible (par exemple la réalisation d'une amplification isothermique à médiation par boucles) ; et
- une deuxième partie 2 constituée par exemple d'un disque perméable, par exemple d'un disque perméable en matière cellulosique, comprenant l'agent intercalant d'ADN.

Un élément chauffant électrique (non illustré) assure un chauffage d'au moins le disque perméable 1 à une température prédéterminée pour assurer l'amplification dudit ADN cible pour obtenir des amplicons de l'ADN cible (par exemple pour assurer une amplification isothermique à médiation par boucles). Selon le mode de réalisation de la figure 6A, des électrodes 3 au nombre de trois sont en contact avec l'échantillon après que ce dernier ait été en contact avec lesdits réactifs permettant la réalisation de l'amplification dudit ADN cible pour obtenir des amplicons de l'ADN cible (par exemple la réalisation d'une amplification isothermique à médiation par boucles), et avec ledit agent intercalant d'ADN. En outre, les électrodes 3 sont également accessibles électriquement depuis l'extérieur du dispositif selon l'invention de telle sorte qu'elles peuvent être reliées électriquement à un appareillage (non illustré) permettant de réaliser des mesures électrochimiques (de préférence des mesures électrochimiques voltampérométriques, plus préférentiellement des mesures électrochimiques voltampérométriques cycliques ou à ondes carrées) utilisant un indicateur redox (agent intercalant), le principe et la mise en œuvre d'une telle mesure (ADN/amplicons + agent intercalant) étant bien connus de l'homme de métier. Comme illustré à la figure 6A, les électrodes 3 du dispositif A selon l'invention peuvent être reliées électriquement à un appareillage (non illustré) par l'intermédiaire d'une base B ou d'un socle muni de connecteurs. Par exemple, le dispositif A selon l'invention peut être visé ou clipsé sur le socle ou la base B pour assurer un contact adéquat entre les connecteurs et les électrodes 3.

La figure 6B est identique à la figure 6A mais une vanne 6, par exemple une vanne à tiroir rotative, sépare la première partie 1 de la deuxième partie 2 et permet de commander le passage de l'échantillon depuis la première partie 1 comprenant les réactifs permettant la réalisation de l'amplification dudit ADN cible pour obtenir des amplicons de l'ADN cible (par exemple la réalisation d'une amplification isothermique à médiation par boucles), vers la deuxième partie 2 comprenant l'agent intercalant d'ADN.

La figure 6C est identique à la figure 6A mais le dispositif A illustré comprend en plus une partie ou zone additionnelle 5 comprenant des réactifs ou une solution pour lyser un échantillon prélevé au départ d'un individu. De la sorte, l'échantillon peut être directement introduit dans le dispositif au niveau de la partie ou zone additionnelle 5 dans laquelle l'échantillon est lysé avant de gagner, par exemple sous l'effet de la gravité, la première partie 1 constituée par exemple d'un disque perméable comprenant les réactifs permettant la réalisation de l'amplification dudit ADN cible pour obtenir des amplicons de l'ADN cible (par exemple la réalisation d'une amplification isothermique à médiation par boucles), puis vers la deuxième partie 2 comprenant l'agent intercalant d'ADN.

La figure 6D est identique à la figure 6C mais le dispositif A illustré comprend en plus une vanne 6', par exemple une vanne à tiroir rotative, permettant de commander le passage de l'échantillon depuis la partie ou zone additionnelle 5 où il est lysé vers la première partie 1 constituée par exemple d'un disque perméable comprenant les réactifs permettant la réalisation de l'amplification dudit ADN cible pour obtenir des amplicons de l'ADN cible (par exemple la réalisation d'une amplification isothermique à médiation par boucles) puis vers la deuxième partie 2 comprenant l'agent intercalant d'ADN.

La figure 6E est identique à la figure 6D mais le dispositif A illustré comprend en plus une vanne 6, par exemple une vanne à tiroir rotative, permettant de commander le passage de l'échantillon depuis la première partie 1 constituée par exemple d'un disque perméable comprenant les réactifs permettant la réalisation de l'amplification dudit ADN cible pour obtenir des amplicons de l'ADN cible (par exemple la réalisation d'une amplification isothermique à médiation par boucles), vers la deuxième partie 2 comprenant l'agent intercalant d'ADN.

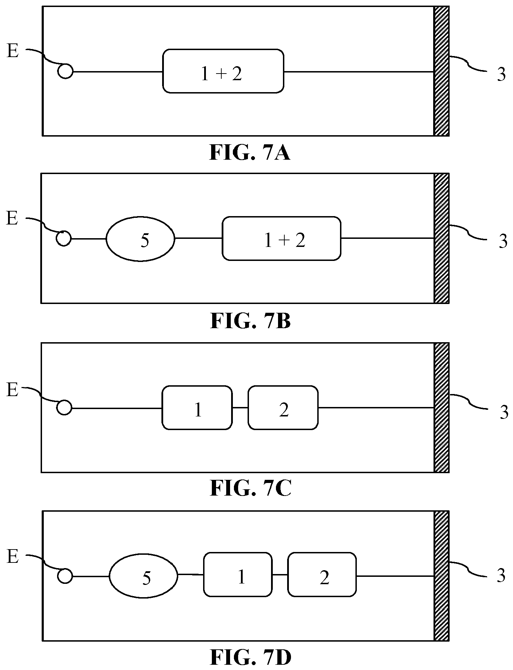

Les figures 7A, 7B, 7C et 7D illustrent des exemples de modes de réalisation de dispositifs selon l'invention sous forme de dispositifs microfluidiques (cellules ou puces microfluidiques) présentant une entrée E via laquelle un échantillon peut être introduit. Des éléments identiques à ceux illustrés aux figures précédentes sont repris.

La présente invention a été décrite en relation avec des modes de réalisations spécifiques, qui ont une valeur purement illustrative et ne doivent pas être considérés comme limitatifs. D'une manière générale, il apparaîtra évident pour l'homme du métier que la présente invention n'est pas limitée aux exemples illustrés et/ou décrits ci-dessus.

L'usage des verbes « comprendre », « inclure », « comporter », ou toute autre variante, ainsi que leurs conjugaisons, ne peut en aucune façon exclure la présence d'éléments autres que ceux mentionnés.

L'usage de l'article indéfini « un », « une », ou de l'article défini « le », « la » ou « l' », pour introduire un élément n'exclut pas la présence d'une pluralité de ces éléments.

## Revendications

1. Dispositif pour détecter la présence d'ADN cible dans un échantillon, en particulier dans un échantillon liquide, ledit dispositif comprenant :
- des réactifs permettant la réalisation d'une amplification dudit ADN cible pour obtenir des amplicons de l'ADN cible, par exemple la réalisation d'une amplification isothermique à médiation par boucles (LAMP) dudit ADN cible pour obtenir des amplicons de l'ADN cible ;
- un agent intercalant d'ADN présentant des propriétés oxydo-réductrices et pouvant s'intercaler entre des molécules desdits amplicons, en particulier pouvant s'intercaler dans un espace compris entre deux paires de bases desdits amplicons ;
- un élément chauffant électrique agencé pour chauffer ledit échantillon pendant que ce dernier est en contact ou après que ce dernier ait été en contact avec lesdits réactifs permettant la réalisation de l'amplification dudit ADN cible pour obtenir des amplicons de l'ADN cible, par exemple la réalisation d'une amplification isothermique à médiation par boucles (LAMP) ; et
- des électrodes agencées pour être en contact avec ledit échantillon pendant que ce dernier est en contact ou après que ce dernier ait été en contact avec lesdits réactifs permettant la réalisation de l'amplification dudit ADN cible pour obtenir des amplicons de l'ADN cible, par exemple la réalisation d'une amplification isothermique à médiation par boucles (LAMP), et avec ledit agent intercalant d'ADN.

2. Dispositif selon la revendication 1, **caractérisé en ce que** lesdites électrodes sont en outre agencées pour être accessibles électriquement depuis l'extérieur dudit dispositif.

3. Dispositif selon la revendication 1 ou 2, **caractérisé en ce qu'**il présente :
- une première partie ou zone comprenant lesdits réactifs permettant la réalisation d'une amplification dudit ADN cible pour obtenir des amplicons de l'ADN cible, par exemple la réalisation d'une amplification isothermique à médiation par boucles (LAMP) ; et
- une deuxième partie ou zone comprenant ledit agent intercalant d'ADN présentant des propriétés oxydo-réductrices et pouvant s'intercaler entre des molécules desdits amplicons.

4. Dispositif selon la revendication 3, **caractérisé en ce qu'**il comprend une vanne située entre ladite première partie ou zone et ladite deuxième partie ou zone.

5. Dispositif selon l'une quelconque des revendications précédentes **caractérisé en ce qu'**il comprend en outre des réactifs pour lyser ledit échantillon ou une solution pour lyser ledit échantillon.

6. Dispositif selon la revendication 5, **caractérisé en ce qu'**il présente une partie ou zone additionnelle comprenant lesdits réactifs pour lyser ledit échantillon ou ladite solution pour lyser ledit échantillon, ladite partie ou zone additionnelle étant située en amont de ladite première partie et de ladite deuxième partie dans un sens d'écoulement/de déplacement de l'échantillon.

7. Dispositif selon la revendication 6, **caractérisé en ce qu'**il comprend une vanne située entre ladite partie ou zone additionnelle et ladite première partie ou zone et/ou ladite deuxième partie ou zone.

8. Dispositif selon l'une quelconque des revendications précédentes, **caractérisé en ce que** lesdits réactifs permettant la réalisation d'une amplification dudit ADN cible pour obtenir des amplicons de l'ADN cible, par exemple la réalisation d'une amplification isothermique à médiation par boucles (LAMP), et/ou ledit agent intercalant d'ADN présentant des propriétés oxydo-réductrices et pouvant s'intercaler entre des molécules desdits amplicons sont sous forme anhydre ou lyophilisée.

9. Dispositif selon l'une quelconque des revendications précédentes, **caractérisé en ce que** ledit agent intercalant et/ou lesdits réactifs permettant la réalisation d'une amplification dudit ADN cible pour obtenir des amplicons de l'ADN cible, par exemple la réalisation d'une amplification isothermique à médiation par boucles (LAMP), sont présents sur un support, par exemple sur un support cellulosique, comportant des charges positives et/ou des groupements chargés positivement et/ou des polymères chargés positivement.

10. Dispositif selon l'une quelconque des revendications précédentes, **caractérisé en ce que** ledit échantillon est un choisi parmi un échantillon sanguin, un échantillon salivaire, un échantillon urinaire.

11. Dispositif selon l'une quelconque des revendications précédentes, **caractérisé en ce que** ledit élément chauffant électrique comprend une résistance électrique.

12. Dispositif selon l'une quelconque des revendications précédentes, **caractérisé en ce que** lesdites électrodes sont au nombre de trois.

13. Dispositif selon l'une quelconque des revendications précédentes, **caractérisé en ce qu'**il se présente sous la forme d'une cartouche ou d'un dispositif microfluidique, par exemple sous forme d'une cellule ou d'une puce microfluidique ou d'un dispositif microfluidique à base de papier.

14. Ensemble comprenant :
- un dispositif selon l'une quelconque des revendications 1 à 13 ; et
- un appareil ou un appareillage pouvant être relié électriquement aux électrodes pour effectuer des mesures électrochimiques, de préférence des mesures électrochimiques voltampérométriques, plus préférentiellement des mesures électrochimiques voltampérométriques cycliques ou à ondes carrées.

15. Utilisation d'un dispositif selon l'une quelconque des revendications 1 à 13 ou d'un ensemble selon la revendication 14 pour détecter la présence d'ADN cible dans un échantillon.
